# EUROPEAN PATENT SPECIFICATION

(11) **EP 1 075 237 B1**
(45) Date of publication and mention of the grant of the patent: **23.03.2005**
(21) Application number: 99947023.0
(22) Date of filing: 27.04.1999
(51) Int. Cl.: A61F 2/64

(54) **KNEE PROSTHESIS**
KNIEPROTHESE
PROTHESE DE L'ARTICULATION DU GENOU

(30) Priority: 28.04.1998 US 83318 P; 13.07.1998 SE 9802515
(43) Date of publication of application: 14.02.2001
(73) Proprietor: GRAMTEC INNOVATION AB, S-511 56 Kinna (SE)
(72) Inventor: GRAMNÄS, Finn, S-511 56 Kinna (SE)
(74) Representative: Egeröd, Lisbeth
(86) International application number: PCT/SE1999/000690
(87) International publication number: WO 1999/055261

(56) References cited:
- US-A- 3 723 997
- US-A- 4 351 070
- US-A- 4 549 318
- US-A- 4 997 449

## Description

The present invention refers to a knee prosthesis as defined in the preamble of claim 1.

### Background of the invention

Traditional friction locks in knee prostheses will lock when there is a load acting on them, i e when some part of the body weight rests on the artificial knee. This takes place in a still standing position as well as during the walking phase at heel strike when the heel hits the ground, and during toe off supporting oneself on the toe while extending the leg to initiate the swing phase when the leg swings freely in the air.

At normal walking without a prosthesis one starts already during toe off to flex the knee-joint to initiate the swing phase before all body weight is has been removed from the leg in question. This is not possible with knee prostheses with a friction lock of the conventional kind. This involves an unnatural walking and makes walking in stairs and broken ground and cycling difficult since the knee prosthesis will lock as soon as it is loaded.

Knee prostheses with friction locks in the form of a brake drum are disclosed in US-A-4,206,519 and 4,351,070. In the last mentioned document there is a linkage transferring motions in the hip axis to the locking device in order to control the locking function thereof in response to the torque of the hip axis.

WO 97/10781 discloses a knee prosthesis as defined in the preamble of claim 1 with a friction lock in the form of a resiliently deformable substantially C-shaped member cooperating with an axle. The C-shaped member can be rotated about the axle in unloaded position, but in a locked position be deformed and locked to the axle.

### The subject and most important features of the invention

It is an object of the invention to provide a knee prosthesis of the kind mentioned above, which permits rotation of the knee-joint in unloaded position and when it still is under body load during toe off when supporting oneself on the toe while extending the leg to initiate the swing phase, but which locks against rotation from extended to flexed position at other loaded positions, i e during heel strike when supporting oneself on the heel and during mid stance when supporting oneself on the whole foot. This has according to the invention been achieved by the features set out in the claims.

A further important advantage of the invention is that the knee-joint has a freewheel effect in such a way that it can always rotate from flexed to extended position also under load. By this for example walking in stairs will be possible. The knee joint will however lock immediately again if it is rotated in the opposite direction, i e from extended to flexed position, under such load that the locking device is activated.

### Description of drawings

The invention will below be described more in detail with reference to some embodiments shown in the accompanying drawings.
Fig. 1 is a longitudinal section through an embodiment of a knee prosthesis according to the invention in unloaded position.
Fig. 2 shows the knee prosthesis according to Fig. 1 in loaded position.
Fig. 3 is an exploded view of the knee prosthesis according to Fig. 1 and 2.
Fig. 4 a-f illustrates the function of the knee prosthesis during the different phases of the walking cycle.
Fig. 5 shows a longitudinal section through a second embodiment of the knee prosthesis in unloaded position.
Fig. 6 is a front view of the knee prosthesis according to Fig.5.
Fig. 7 is an exploded view of the knee prosthesis according to Fig. 5 and 6.

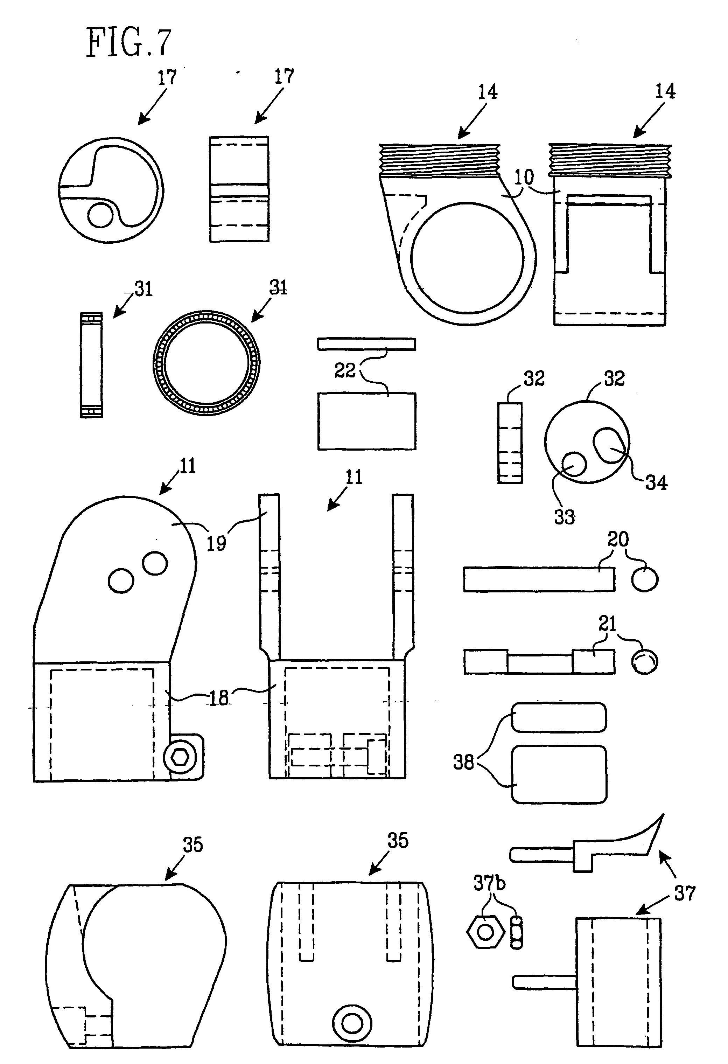

Fig. 7 is a longitudinal section through a third embodiment of the knee prosthesis.

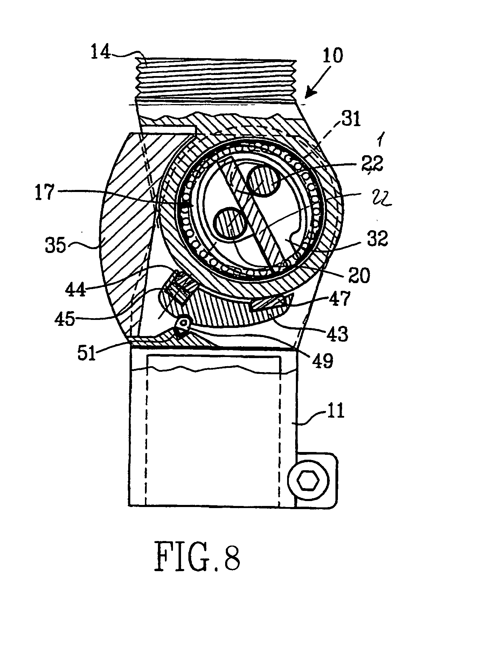

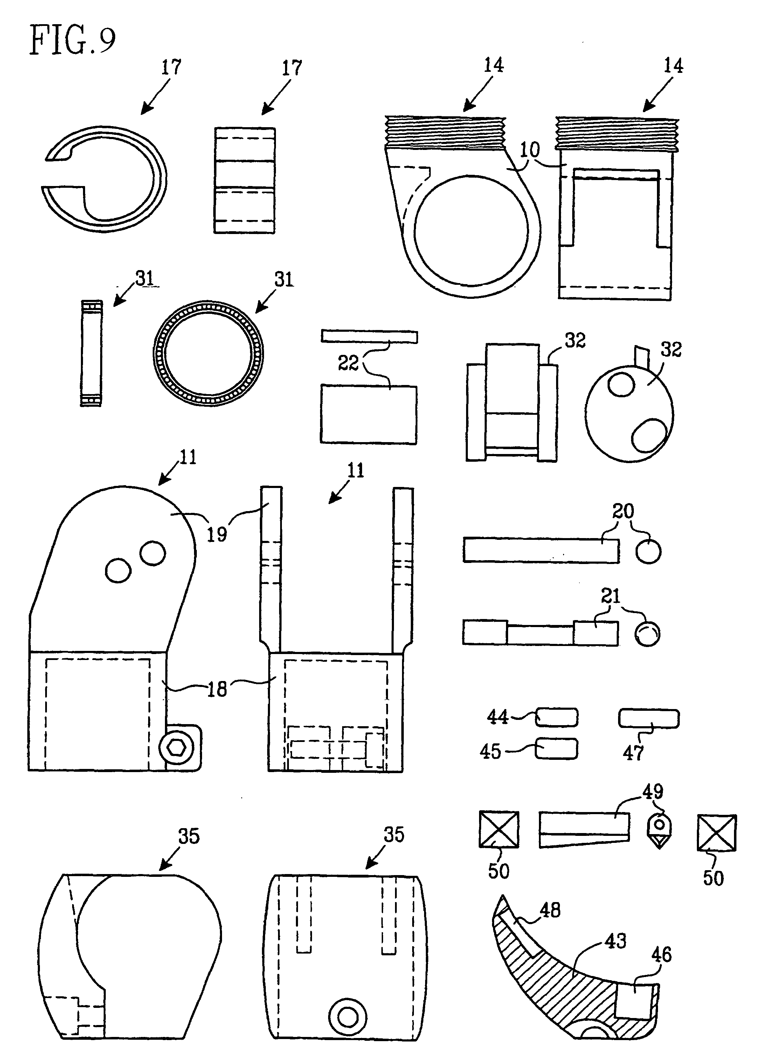

Fig. 8 is an exploded view of the knee prosthesis according to Fig. 7.

### Description of embodiments

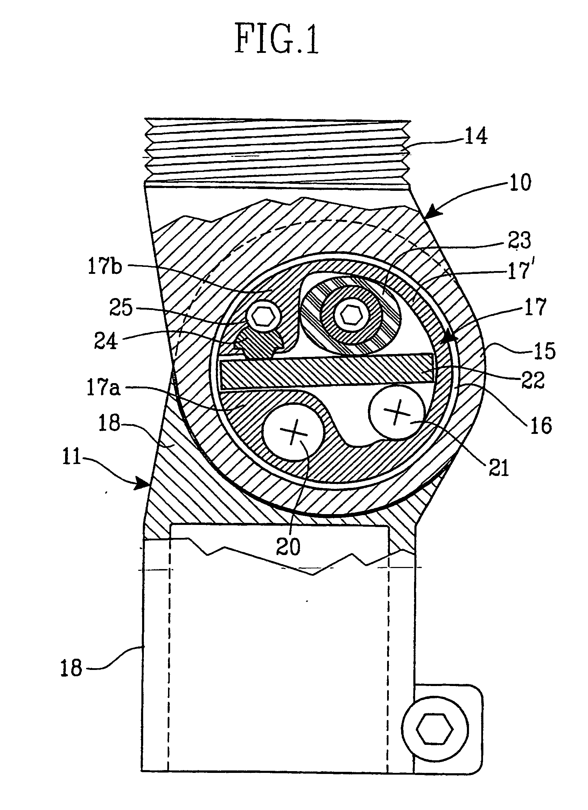

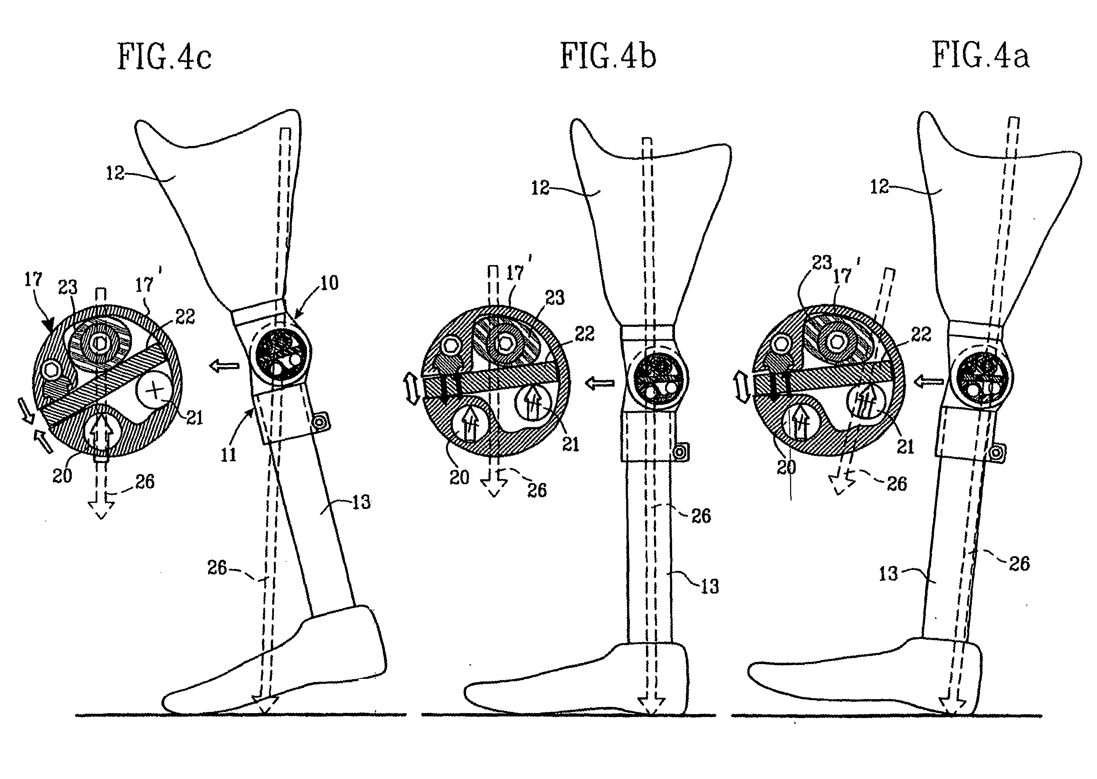

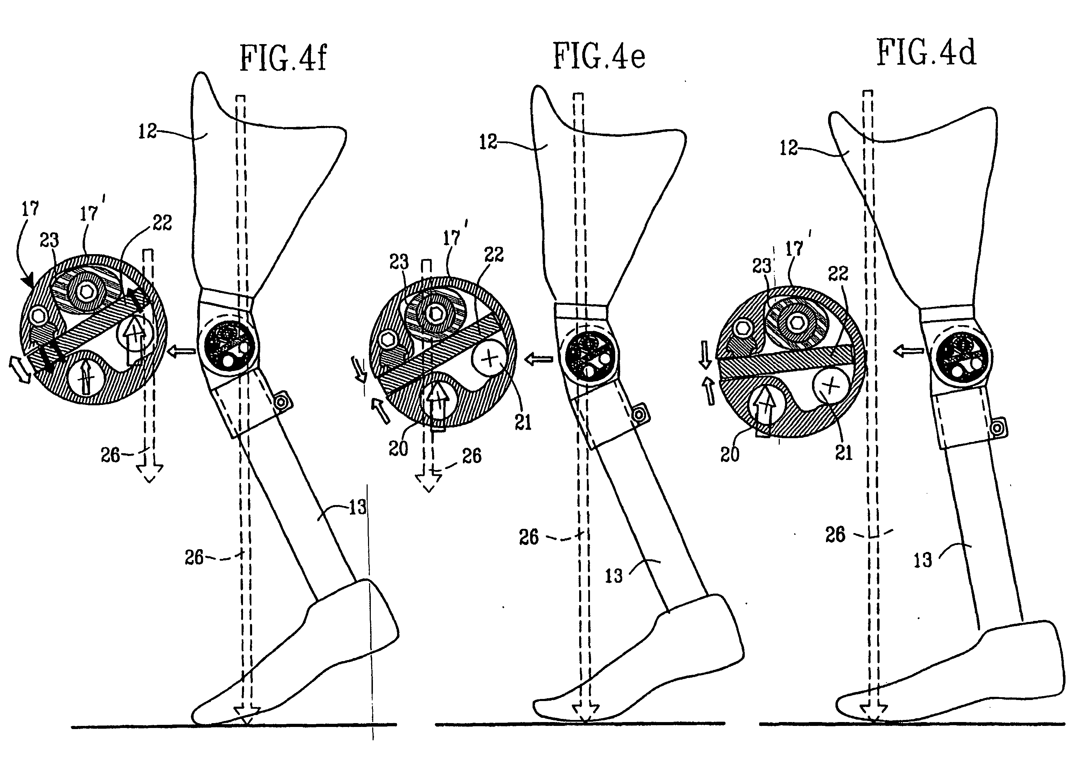

The knee prosthesis according to the embodiment shown in Fig. 1-4 comprises an upper and a lower member 10 and 11 which are pivotally interconnected. The upper member is intended to be attached to a prosthesis sleeve 12 and the lower member to a lower leg prosthesis 13 (Fig. 4) having a foot prosthesis 13a connected thereto.

The upper knee prosthesis member 10 comprises a socket, in the shown embodiment a threaded socket 14, for connection to a prosthesis sleeve, and a housing portion 15 with a through opening 16 for receiving a locking device 17 in the form of a brake drum 17'. The lower knee prosthesis member 11 is provided with a sleeve-shaped socket 18 for connection to a lower leg prosthesis 13, and a yoke-shaped upper portion 19 between the shanks 19 a and b of which the housing portion 15 of the upper knee prosthesis member 10 is received.

Two axles 20 and 21 extend substantially in parallel through the shanks 19 and the brake drum 17' arranged in the housing portion 15. The brake drum 17' is pivotally mounted about the first axle 20. The second axle 21 passes right through the open space in the brake drum 17' and forms a part of the activation mechanism for the braking function of the knee prosthesis in a way which will be described more in detail below. The second axle 21 is arranged at a certain distance behind the first axle 20.

In the braking mechanism there is included besides the brake drum 17' an activating member 22 in the form of a lever arm extending through the brake drum 17' substantially across the axles 20 and 21. The brake drum17' has the shape of an open ring and the lever arm 22 extends in between the shanks 17a and b of the brake drum. The lever arm 22 supports against the second axle 21 and can thereby be pressed upwards and in such a way force the shanks 17 a and b to spring apart, at which the brake drum 17' expands and is friction locked against the inside of the opening 16. Between the lever arm 22 and the brake drum 17' on the opposite side of the lever arm with respect to the second axle 21 there is arranged an adjustable resilient element 23 against which the lever arm presses when it is activated by the second axle 21, said resilient element 23 thus limiting the action of the lever arm 22. The resilient element 23, which comprises a sleeve through which a screw 23a extends, can be made stiffer in order to adapt to the body weight of the wearer of the prosthesis and thus prevent that the knee-joint will lock too easily. The stiffness of the element 23 can be adjusted either by tightening the screw 23a at which the sleeve is compressed, or by replacing the element 23 for stiffer or softer rubber element 23.

The lever arm 22 can optionally be made resilient to some extent, e g be made of a number of spring steel elements placed on top of each other, at which it will be possible to accomplish the light resilient knee flexion one normally makes when putting the heel in the ground, so called stans flex. The element 23 must in this case be relatively soft.

One shank 17a of the brake drum 17' holds a bearing for the first axle 20 while the second shank 17b supports friction adjustment means 24,25 for controlling the friction level during the different phases of the walking cycle. These friction adjustment means comprises a conical wedge 25 and a screw 24 cooperating therewith. By tightening the screw 24 the wedge 25 is moved and can expand the brake drum 17'. A friction adjustment could alternatively be done by squeezing together the shanks 19a and b of the yoke-shaped part 19. A brake lining, e g a plastic band, could be arranged on the outside of the brake drum.

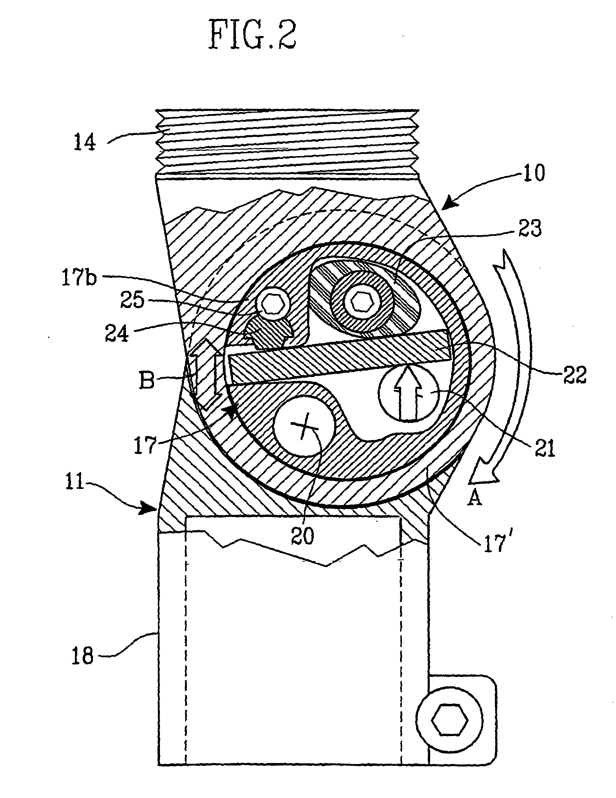

In Fig.1 the knee-joint is shown in a position where the brake drum 17' is unloaded and thus can be rotated within the opening 16 of the housing portion 15. If however a load (Fig. 2) is applied on the leg the brake drum 17' will be pressed downwards and at the same time rotate about the first axle 20 in the direction of the arrow A. When the brake drum is rotated about the axle 20 the rear brake-activation axle 21 will meet the lever arm 22, which as described above forces the brake drum 17' to expand (arrow B) and lock against the inside of the opening 16 in the housing portion 15. The knee joint is by this locked.

If the knee joint is rotated from flexed to extended position, i e in the opposite direction with respect to the arrow A, during loading, the motion between the inside of the opening 16 in the housing portion 15 and the brake drum 17' will force this to rotate back to the position for unloaded position (dashed lines). The lever arm 22 will then leave the axle 21 and the knee joint can be rotated freely in counter clockwise direction. It will however immediately lock again if it during loading will be rotated in clockwise direction.

In Fig. 4 a-f the braking function of the knee prosthesis is shown during the different phases of the walking cycle. In Fig. 4a the heel strike position is shown, in which the heel strikes the ground and the wearer starts to put load on the leg in question. The body weight line illustrated by the arrow 26 in this position passes right through the second axle 21 or behind this, which means that the whole body weight will force the brake drum 17' to rotate downwards about the axle 20. The lever arm 22 will then meet the axle 21, which in turn presses against the lever arm 22 which forces the brake drum 17' to expand and lock the knee joint prosthesis. If the bending force would increase the knee joint will use the front axle 20 as a fixed point and the distance between the axles 20 and 21 will act as a lever arm and increase the braking force proportionally to the increasing bending force. This will keep the knee locked independently of the bending forces which act upon the knee joint.

In Fig. 4b is shown the mi stance position when the whole foot rests against the ground and the body weight acts essentially right through the leg in parallel therewith. The body weight line 26 passes in this position between the two axles 20 and 21, which means that both the front 20 and the rear axle 21 are loaded, at which the latter still presses against the lever arm 22 and locks the brake drum 17'. If the prosthesis wearer in this position would try to flex the knee joint this would only lead to an increased force on the rear brake-activating axle 21 in the way described above.

In Fig. 4c is shown the toe off phase when supporting oneself on the toe while extending the leg to initiate the swing phase with the leg swinging freely in the air. A part of the body weight rests in this position still upon the leg. At normal walking a flexing of the knee joint takes is initiated already in this position in order to initiate the swing phase before all body weight has been removed from the leg in question. The body weight line 26 passes in this position from the toe part of the foot prosthesis 13a through or in front of the front axle 20, the bearing axle, at which the body weight rests on the front axle 20. No load acts on the rear brake-activation axle 21, at which the knee joint is free to rotate for initiating the swing phase of the walking cycle.

In Fig. 4d-f are shown different stages of the initiation of the swing phase of the walking cycle with the knee joint prosthesis in different positions. In Fig. 4d is shown an extended leg in toe off position at which the body weight line 26 passes from the toe portion of the foot prosthesis 13a and in front of the front axle 20. In a corresponding way as described in connection with Fig. 4c the brake function is inactivated despite that body weight is still loading the leg and the knee joint can be flexed for initiating the swing phase.

Fig. 4e shows the toe off position with the knee flexed approximately 45°. Most of the body weight has now been removed from the leg, but there can still be enough load for activating the brake function. However the body weight line 26 will still be in front of the front axle 20, and no load will act upon the rear brake-activation axle 21. If however the prosthesis wearer in this position would stumble the body weight can be moved backwards by extending the thighbone and by that have the brake activated.

In Fig. 4f is shown the end of the toe off phase just as the leg has been unloaded from the body weight. The leg is now flexed approximately 55 ° and the body weight line 26 passes through the rear brake-activating 21, but since the leg is unloaded the knee joint can be rotated. If however the prosthesis wearer in this position would stumble or something else unexpected would happen the prosthesis wearer can activate the brake by extending the thighbone.

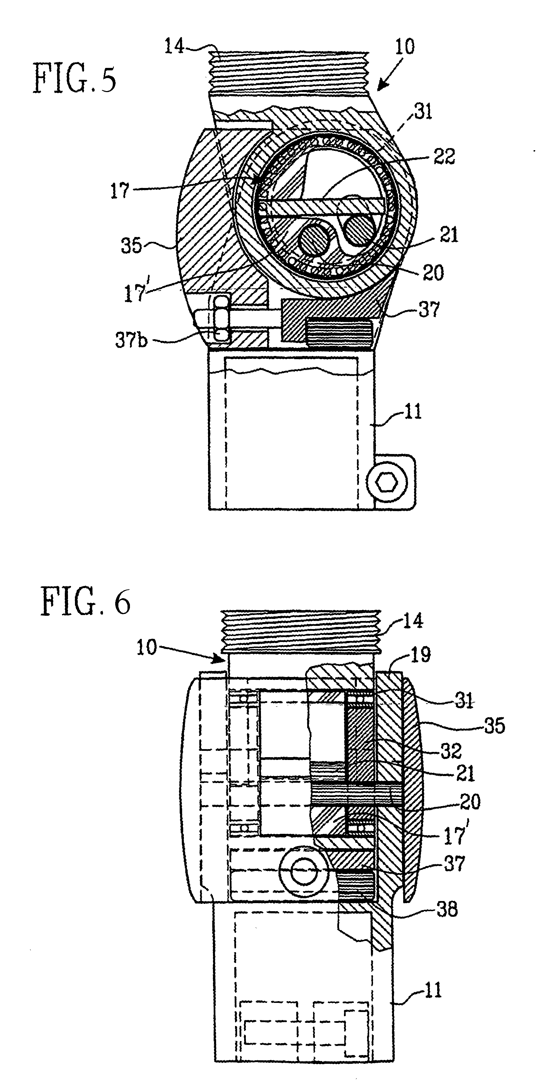

The embodiment according to Fig. 5-7 is similar to the one shown in Fig. 1-4 and differs mainly therefrom by the fact that the brake drum 17' has been made shorter in order to make space for a ball bearing 31 on both sides. By this the lateral stability of the knee joint is improved. The stabilization of the ball bearings 31 is transferred via a plate 32 placed in the centre of the bearings, said plate having a hole 33 for the front axle 20 and an oblong hole 34 for permitting the brake-activating movement of the second axle 21. The resilient element 23 in the embodiment according to Fig. 1-4 has been replaced by a functionally corresponding member 38. The friction adjusting means 24-25 according to Fig. 1-4 has been replaced by the parts 35, 37 and 39.

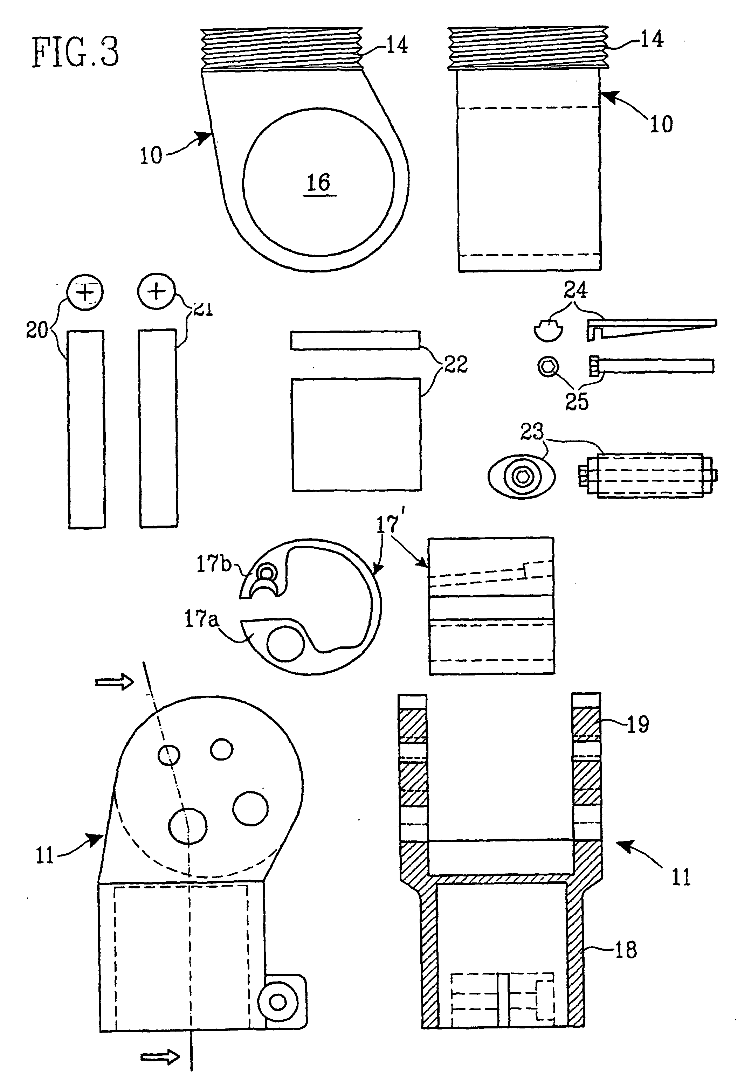

The upper knee joint member 10 has a lower part-cylindrical outer surface 10a. The housing member 35 is attached to the lower knee joint member 11 by means of the adjusting means 37 and the nut 37b. The resilient element 38 is placed under the adjusting means 37 and will assist this to press the upper knee joint member 10 and its part-cylindrical surface 10a against the housing portion 35 and its corresponding surface 35a. If the nut 37b is tightened the adjusting means 37 will press harder against the resilient element 38 since this is forced to slide. downwards along the part-cylindrical surface 10a. The upper knee joint member 10 will then be clamped between the adjusting means 37 and the housing portion 35 actuated by the compressed resilient element 38. This gives a frictional resistance as the knee joint member 10 is rotated. The inner brake drum is now not needed for braking the swing movement of the leg but only be used for locking the knee joint. The resilient element 38 has also the same function as the element 23 in Fig. 3. In order to load the brake drum 17' and the upper knee joint member 10 until a rotation is initiated about the first axle 20 and activate the brake, the resilient element 38 has to becompressed via the adjusting means 37 in the movement about the first axle 20.

The embodiment according to Fig. 7 and 8 is similar to the one disclosed in Figs. 5-7. However the construction of the brake drum 17 and of the plates 32 differ. The plates 32 which are provided with holes 33 and 34 for the axles 20 and 21 are interconnected by an intermediate member 32' around which the brake drum 17 is arranged. The brake drum 17 in this case has no hole for the axle 20.

There is an essential difference in the construction of the friction adjustment device, with which the friction resistance against rotation of the knee joint is adjusted. This device comprises in this case a brake shoe 43, which can be tightened against the part-cylindrical surface 10a of the upper knee joint member 10. A friction element 44 which rests against a resilient rubber element 45 is arranged in a first recess 46 in the brake shoe 43, while a sliding block 47, e g by Teflon, is placed in a second recess 48 at the opposite end of the brake shoe. The brake shoe 43 is so arranged with respect to the axles 20 and 21 that the friction element 44 will press against the knee joint member 10 at a point just opposite the axle 20 and its connection line with the axle 21. By this the important advantage is obtained that the friction in the knee joint can be adjusted without in a corresponding way influencing the function of the locking mechanism, i e the axle 21, the lever arm 22 and the brake drum 17.

The friction adjusting device further includes a conical adjusting member 49, which by means of adjusting screws 50 can be displaced in a wedge-shaped groove 51 in the housing portion 35. By displacing the adjusting member 49 in the groove 51 the contact pressure of the friction element 44 against the knee joint member 10 is increased or decreased. In the shown embodiment the distance between the adjusting member 49 and the friction element 44 is about one third of the distance between the adjusting member 49 and the sliding block 47. This means that when a lifting force is applied on the adjusting member 49 by displacing this in its groove 51, this lifting force will be distributed so that 75% will be applied on the friction element 44 and 25% on the sliding block 47. The part of the lifting force from the adjusting member 49 which will be applied on the sliding block 47 is used for creating an upwards directed lifting force on the knee joint member 10. By this an unintentional locking of the brake drum is prevented 10.

By this arrangement there is required a smaller force at heel strike for activating the brake drum 17 as compared to the constructions shown above, where e g in Fig. 2 the entire expansion force from the element has to be overcome in order to get the brake to lock. The same thing applies also to Fig. 8 where the entire force from the resilient element has to be overcome before the brake locks. In the embodiment according to Fig. 14 there is only required that the lifting force from the sliding block 47 is overcome, said force amounts only to 25% of the total lifting force applied on the knee joint member 10 by means of the adjusting member 49, the brake shoe 43 and the friction elements 44 and 47.

The above described principle for frictional adjustment of the rotation of the knee joint, where the frictional pressure applied on the knee joint member 10 just opposite the axle 20 and its imaginary connection line with the brake-activating axle 21, may of course be designed in different ways than shown in Figs. 7 and 8, and can also be applied to the other embodiments. The advantage is as disclosed above that the frictional resistance against the rotation of the knee joint acts and can be adjusted less dependant on the activation of the locking mechanism.

In all above described embodiments the relative placement of the two axles 20 and 21 is important for achieving the desired locking effect. Thus the first bearing axle 20 and the. second brake-activating axle 21 should be located in such a position with respect to each other that the line of action 26 from a loading of the knee prosthesis in connection with toe off during the walking cycle will pass through the first axle 20 or in front thereof, so that the second axle 21 is unloaded and the locking device 17 inactivated. By this the knee prosthesis will only lock at heel strike and at mid stance while supporting oneself with the whole foot on the ground, while rotation is admitted when the knee joint is still loaded at toe off.

All described embodiments have a freewheel effect in such a way that the knee joint can always rotate from flexed to extended position also under load. By this for example walking in stairs will be possible. The knee-joint will however lock immediately again if it is rotated in the opposite direction, i e from extended to flexed position, under such load that the locking device is activated.

The invention is of course not limited to the embodiments shown in the drawings but can be modified within the scope of the following claims. It applies for example for all embodiments that the coupling means to the prosthesis sleeve and to the lower leg can be of another type than described herein.

## Claims

1. A knee prosthesis comprising two pivotally interconnected members (10,11) carrying a locking device (17) and a first (20) and a second axle (21), said second axle being located at a distance behind the first axle as seen in the direction of walking, said locking device is arranged to permit said members to pivot in an unloaded position but prevent pivoting from extended to bent position when loaded,
the locking device (17) comprises a brake drum (17'), which by means of the first axle (20) is pivotally connected to one of said knee prosthesis members (11) **characterized in that** the brake drum (17') cooperates with the second axle (21) in such a way that when the second axle is loaded the brake drum (17') will expand and frictionally lock within the second knee prosthesis member (10) designed as a bearing housing, at which the two knee prosthesis members (10,11) will lock to each other, while when the second axle (21) is unloaded the two knee prothesis members (10, 11) can rotate with respect to each other, wherein the structural relationship between the first and second axles (21 and 22) being such that when the line of action (26) from a load on the knee prosthesis passes through the second axle (21) or between the first and second axles, said second axle (21) it will act upon the brake drum (17') to expand and activate it, while when the line of action (26) from said load passes through the first axle (20) or on a side of it which faces away from the second axle (21) the second axle (21) will be unloaded and the brake drum (17') inactivated.

2. A knee prosthesis according to claim 1,
**characterized in**
**that** the second axle (21) cooperates with a lever arm (22) which activates the brake drum (17') against the action of a resilient element (23) arranged between the brake drum and the lever arm on the opposite side thereof with respect to the second axle (21).

3. A knee prosthesis according to claim 2,
**characterized in**
**that** the resilient element (23) is adjustable for adapting to the body weight of the prosthesis wearer.

4. A knee prosthesis according to any of claims 1-3,
**characterized in**
**that** the brake drum (17') has the shape of an open ring and that the lever arm (22) extends between the shanks (17a,b) forming the opening of the ring and at activation of the brake drum forces said shanks apart.

5. A knee prosthesis according to claim 4,
**characterized in**
**that** the first axle (20) is mounted in one shank (17a) of the brake drum, while the second shank (17b) supports friction adjusting means (24,25).

6. A knee prosthesis according to claim 1,
**characterized in**
**that** the locking device comprises a brake drum (17') in the form of an open ring one end surface of which cooperates with the second axle (2 1 ).

7. A knee prosthesis according to any of the preceding claims,
**characterized in**
**that** the knee prosthesis has a freewheel effect in such a way that the knee joint can always rotate from flexed to extended position also under load.

8. A knee prosthesis according to any of the preceding claims,
**characterized in**
**that** a friction brake (43,44) is arranged to act upon the first knee joint member (10) for braking the rotational movement thereof relative the second knee joint member (11), said friction brake is arranged to act upon the first knee joint member (10) at a point located just opposite the first axle (20) and its connection line with the second axle (21).

9. A knee prosthesis according to claim 8,
**characterized in**
**that** the friction brake is designed as a brake shoe (43) which at one end carries a friction element (44) arranged to create friction against the first knee joint member (10) for braking the rotational movement thereof relative to the second knee joint member (11) and at its other end carries a sliding element (47) arranged to create a lifting force on the first knee joint member (10) for preventing it to unintentionally activate the locking device (17).

10. A knee prosthesis according to claim 9,
**characterized in**
**that** an adjusting device (49,50,51) is arranged to act on the brake shoe (43) for adjusting the contact pressure of the friction element (44) and the sliding element (47) against the first knee joint member (10), at which the adjustment device (49,50,51) is arranged to act on a point located at a shorter distance from the friction element (44) than from the sliding element (47), so that when tightening the adjusting element a larger part of the contact pressure will be distributed to the friction element (44) than to the sliding element (47).

11. A knee prosthesis according to any of the preceding claims having a lower leg and foot prosthesis connected to the knee prosthesis,
**characterized in**
**that** the first and second axle (20,21) are located at such a mutual distance that the line of action (26) from a loading of the knee prosthesis in connection with toe off of the foot prosthesis during the walking cycle, will pass with respect to the first and second axles (20,21), so that the second axle (21) is unloaded and the locking device (17) inactivated.

## Patentansprüche

1. Eine Knieprothese, die zwei schwenkbar miteinander verbundene Elemente (10, 11) mit einer Verschlussvorrichtung (17) und einen ersten (20) und einen zweiten Schaft (21) aufweist, wobei sich der zweite Schaft in einem Abstand hinter dem ersten Schaft, in Gehrichtung gesehen, befindet und die Verschlussvorrichtung so angeordnet ist, dass die Elemente in einer unbelasteten Position schwenken können, jedoch das Schwenken von einer gestreckten zur gebeugten Position bei Belastung verhindert wird, wobei die Verschlussvorrichtung (17) eine Bremstrommel (17') umfasst, welche mit Hilfe des ersten Schafts (20) schwenkbar mit einem der Knieprothesenelemente (11) verbunden ist,
**dadurch gekennzeichnet, dass**
die Bremstrommel (17') mit dem zweiten Schaft (21) dergestalt zusammenwirkt, dass, wenn der zweite Schaft belastet wird, sich die Bremstrommel (17') ausdehnt und reibschlüssig im zweiten Knieprothesenelement (10) in Eingriff kommt, das als Lagergehäuse ausgebildet ist, wobei die zwei Knieprothesenelemente (10, 11) miteinander in Eingriff kommen, während, wenn der zweite Schaft (21) unbelastet ist, sich die zwei Knieprothesenelemente (10, 11) in Bezug aufeinander drehen können, wobei das strukturelle Verhältnis zwischen dem ersten und dem zweiten Schaft (21 und 22) dergestalt ist, dass, wenn die Aktionslinie (26) von einer Belastung auf der Knieprothese durch den zweiten Schaft (21) oder zwischen dem ersten und dem zweiten Schaft hindurch verläuft, der zweite Schaft (21) auf die Bremstrommel (17') einwirkt, um diese zu erweitern und zu betätigen, während, wenn die Aktionslinie (26) von dieser Belastung durch den ersten Schaft (20) hindurch oder an einer Seite desselben entlang verläuft, die vom zweiten Schaft (21) abgewandt ist, der zweite Schaft (21) entlastet wird und die Bremstrommel (17') deaktiviert wird.

2. Eine Knieprothese nach Anspruch 1,
**dadurch gekennzeichnet, dass**
der zweite Schaft (21) mit einem Hebelarm (22) zusammenwirkt, welcher die Bremstrommel (17') gegen die Wirkung eines elastischen Elements (23) betätigt, das zwischen der Bremstrommel und dem Hebelarm auf der gegenüberliegenden Seite desselben in Bezug auf den zweiten Schaft (21) angeordnet ist.

3. Eine Knieprothese nach Anspruch 2,
**dadurch gekennzeichnet, dass**
das elastische Element (23) für die Anpassung an das Körpergewicht des Prothesenträgers verstellbar ist.

4. Eine Knieprothese nach einem der Ansprüche 1 bis 3,
**dadurch gekennzeichnet, dass**
die Bremstrommel (17') die Form eines offenen Rings hat und dass sich der Hebelarm (22) zwischen den Schenkeln (17 a, b) erstreckt, welche die Öffnung des Rings bilden und bei Betätigung der Bremstrommel diese Schenkel auseinander drückt.

5. Eine Knieprothese nach Anspruch 4,
**dadurch gekennzeichnet, dass**
der erste Schaft (20) in einem Schenkel (17a) der Bremstrommel angebracht ist, während der zweite Schenkel (17b) Reibungsanpassungsvorrichtungen (24, 25) trägt.

6. Eine Knieprothese nach Anspruch 1,
**dadurch gekennzeichnet, dass**
die Verschlussvorrichtung eine Bremstrommel (17') in Form eines offenen Rings aufweist, dessen eine Endoberfläche mit dem zweiten Schaft (21) zusammenwirkt.

7. Eine Knieprothese nach einem der vorangehenden Ansprüche,
**dadurch gekennzeichnet, dass**
die Knieprothese einen Freilaufeffekt aufweist, dergestalt, dass sich das Kniegelenk immer, auch unter Belastung, von der gebeugten in die gestreckte Position drehen kann.

8. Eine Knieprothese nach einem der vorangehenden Ansprüche,
**dadurch gekennzeichnet, dass**
eine Reibungsbremse (43, 44) zur Einwirkung auf das erste Kniegelenkelement (10) angeordnet ist, um die Drehbewegung desselben relativ zum zweiten Kniegelenkelement (11) zu bremsen, wobei die Reibungsbremse zur Einwirkung auf das erste Kniegelenkelement (10) an einem Punkt angeordnet ist, der sich gegenüber dem ersten Schaft (20) und seiner Verbindungslinie mit dem zweiten Schaft (21) befindet.

9. Eine Knieprothese nach Anspruch 8,
**dadurch gekennzeichnet, dass**
die Reibungsbremse als Bremsbacke (43) ausgebildet ist, welche an einem Ende ein Reibungselement (44) trägt, das so angeordnet ist, dass es Reibung gegen das erste Kniegelenkelement (10) erzeugt, um die Drehbewegung desselben relativ zum zweiten Kniegelenkelement (11) zu bremsen, und an seinem anderen Ende ein Gleitelement (47) trägt, das so angeordnet ist, dass es eine Hebekraft auf dem ersten Kniegelenkelement (10) erzeugt, um dieses daran zu hindern, unabsichtlich die Verschlussvorrichtung (17) zu betätigen.

10. Eine Knieprothese nach Anspruch 9,
**dadurch gekennzeichnet, dass**
eine Einstellvorrichtung( 49, 50, 51) angeordnet ist, um auf die Bremsbacke (43) einzuwirken, um den Kontaktdruck des Reibungselements (44) und des Gleitelements (47) gegen das erste Kniegelenkelement (10) anzupassen, an dem die Einstellvorrichtung (49, 50, 51) so angeordnet ist, dass sie an einem Punkt einwirkt, der sich in einem kürzeren Abstand vom Reibungselement (44) als vom Gleitelement (47) befindet, so dass beim Festziehen des Einstellelements ein größerer Teil des Kontaktdrucks auf das Reibungselement (44) als auf das Gleitelement (47) verteilt wird.

11. Eine Knieprothese nach einem der vorangehenden Ansprüche mit einer mit der Knieprothese verbundenen Unterschenkel- und Fußprothese,
**dadurch gekennzeichnet, dass**
der erste und zweite Schaft (20, 21) sich in einem solchen Abstand voneinander befinden, dass die Aktionslinie (26) von einer Belastung der Knieprothese in Verbindung mit dem Anheben der Zehen der Fußprothese während des Gehzyklus in Bezug auf den ersten und zweiten Schaft (20, 21) so verläuft, dass der zweite Schaft (21) entlastet wird und die Verschlussvorrichtung (17) deaktiviert wird.

## Revendications

1. Prothèse de genou comportant deux éléments connectés mutuellement de manière pivotante (10, 11) supportant un dispositif de verrouillage (17) et un premier essieu (20) et un second essieu (21), ledit second essieu étant positionné à une distance derrière le premier essieu lorsque vu dans la direction de marche, ledit dispositif de verrouillage étant agencé pour permettre auxdits éléments de pivoter dans une position déchargée mais pour empêcher un pivotement à partir d'une position étendue vers une position incurvée lorsqu'ils sont chargés, le dispositif de verrouillage (17) comportant un tambour de frein (17') qui, par l'intermédiaire du premier essieu (20), est connecté de manière pivotante à l'un desdits éléments de prothèse de genou (11), et
**caractérisée en ce que** le tambour de frein (17') coopère avec le second essieu (21) d'une manière telle que lorsque le second essieu est chargé, le tambour de frein (17') va se déployer et se verrouiller avec frottement dans le second élément de prothèse de genou (10) conçu comme un boîtier de palier, où les deux éléments de prothèse de genou (10, 11) vont se verrouiller l'un avec l'autre, tandis que lorsque le second essieu (21) est déchargé, les deux éléments de prothèse de genou (10, 11) peuvent tourner l'un par rapport à l'autre, la relation structurelle entre les premier et second essieux (21, 22) étant telle que lorsque la ligne d'action (26) à partir d'une charge sur la prothèse de genou passe à travers le second essieu (21) ou entre les premier et second essieux, ledit second essieu (21) va agir sur le tambour de frein (17') pour le déployer et l'activer, tandis que lorsque la ligne d'action (26) à partir de ladite charge passe à travers ledit premier essieu (20) ou sur un côté de celui-ci qui est dirigé à l'opposé du second essieu (21), le second essieu (21) va être déchargé, et le tambour de frein (17') va être désactivé.

2. Prothèse de genou selon la revendication 1,
**caractérisée en ce que** le second essieu (21) coopère avec un bras de levier (22) qui active le tambour de frein (17') à l'encontre de l'action d'un élément élastique (23) agencé entre le tambour de frein et le bras de levier sur le côté opposé de celui-ci par rapport au second essieu (21).

3. Prothèse de genou selon la revendication 2,
**caractérisée en ce que** l'élément élastique (23) est ajustable pour adaptation au poids de corps de l'utilisateur de la prothèse.

4. Prothèse de genou selon l'une quelconque des revendications 1 à 3,
**caractérisée en ce que** le tambour de frein (17') a la forme d'un anneau ouvert, et **en ce que** le bras de levier (22) s'étend entre les queues (17a, b) formant l'ouverture de l'anneau et, à l'actionnement du tambour de frein, pousse lesdites queues loin l'une de l'autre.

5. Prothèse de genou selon la revendication 4,
**caractérisée en ce que** le premier essieu (20) est monté dans une première queue (17a) du tambour de frein, tandis que la seconde tige (17b) supporte des moyens d'ajustement de frottement (24, 25).

6. Prothèse de genou selon la revendication 1,
**caractérisée en ce que** le dispositif de verrouillage comporte un tambour de frein (17') ayant la forme d'un anneau ouvert dont une surface d'extrémité coopère avec le second essieu (21).

7. Prothèse de genou selon l'une quelconque des revendications précédentes,
**caractérisée en ce que** la prothèse de genou a un effet de roue libre de telle manière que l'articulation du genou peut toujours tourner à partir de la position fléchie vers la position étendue, également sous une charge.

8. Prothèse de genou selon l'une quelconque des revendications précédentes,
**caractérisée en ce qu'**un frein à frottement (43, 44) est agencé pour agir sur le premier élément d'articulation de genou (10) pour freiner son déplacement de rotation par rapport au second élément d'articulation de genou (11), ledit frein à frottement étant agencé pour agir sur le premier élément d'articulation de genou (10) au niveau d'un point positionné juste opposé au premier essieu (20) et a sa ligne de connexion avec le second essieu (21).

9. Prothèse de genou selon la revendication 8,
**caractérisée en ce que** le frein à frottement est conçu sous la forme d'un patin de frein (43) qui, à une première extrémité, supporte un élément de frottement (44) agencé pour créer un frottement contre le premier élément d'articulation de genou (10) afin de freiner son déplacement de rotation par rapport au second élément d'articulation de genou (11), et au niveau de son autre extrémité supporte un élément coulissant (17) agencé pour créer une force de levage sur le premier élément d'articulation de genou (10) pour l'empêcher d'activer le dispositif de verrouillage (17) de manière non-voulue.

10. Prothèse de genou selon la revendication 9,
**caractérisée en ce qu'**un dispositif d'ajustement (49, 50, 51) est agencé pour agir sur le patin de frein (43) pour ajuster la pression de contact de l'élément de frottement (44) et de l'élément coulissant (47) contre le premier élément d'articulation de genou (10), au niveau de laquelle le dispositif d'ajustement (49, 50, 51) est agencé pour agir sur un point positionné à une distance plus courte à partir de l'élément de frottement (44) qu'à partir de l'élément coulissant (47), de sorte que lors du serrage de l'élément d'ajustement, une partie plus importante de pression de contact va être répartie vers l'élément de frottement (44) plutôt que vers l'élément coulissant (47).

11. Prothèse de genou selon l'une quelconque des revendications précédentes, ayant une partie de jambe inférieure et une prothèse de pied connectée à la prothèse de genou,
**caractérisée en ce que** les premier et second essieux (20, 21) sont positionnés à une telle distance mutuelle que la ligne d'action (26), à partir d'une charge de la prothèse de genou en association avec les orteils à l'extérieur de la prothèse de pied pendant le cycle de marche, va passer par rapport aux premier et second essieux (20, 21), de sorte que le second essieu (21) est déchargé et le dispositif de verrouillage (17) est désactivé.
